## Europäisches Patentamt

## European Patent Office

## Office européen des brevets

(11) Publication number: **0 034 231**
**B1**

(12) **EUROPEAN PATENT SPECIFICATION**

(45) Date of publication of patent specification: **26.09.84**

(51) Int. Cl.³: **A 61 M 5/28,** A 61 M 5/32

(21) Application number: **80300473.8**

(22) Date of filing: **19.02.80**

(54) Hypodermic syringes.

(43) Date of publication of application:
**26.08.81 Bulletin 81/34**

(45) Publication of the grant of the patent:
**26.09.84 Bulletin 84/39**

(84) Designated Contracting States:
**AT BE CH DE FR GB IT LU NL SE**

(56) References cited:
**WO-A-79/00239**
**DE-U-7 425 713**
**FR-A-2 220 280**
**FR-A-2 342 079**
**US-A-3 320 954**
**US-A-3 605 744**
**US-A-3 796 359**
**US-A-3 797 489**
**US-A-3 797 491**
**US-A-3 890 971**
**US-A-3 893 608**
**US-A-4 188 950**

(73) Proprietor: **Wardlaw, Stephen Clark**
**128 Sunset Hill Drive**
**Branford, Connecticut 06405 (US)**

(72) Inventor: **Wardlaw, Stephen Clark**
**128 Sunset Hill Drive**
**Branford, Connecticut 06405 (US)**

(74) Representative: **Thomas, Christopher Hugo**
**et al**
**D Young & Co 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to hypodermic syringes, and more particularly, but not exclusively, to disposable hypodermic syringes.

There is a need in the medical field for a disposable hypodermic syringe which can be factory pre-loaded with an accurately measured amount of medicament and then safely store the medicament for an extended period of time, say one year or more, before use. This type of syringe could be used by both trained and untrained persons, and if made to operate automatically, could be used by laymen to self-administer injections, or to administer injections to others.

One problem to be solved with such syringes and which also arises with non-disposable syringes, relates to the needle which is left projecting from the syringe after it has been used and which may cause injury to someone handling the syringe. One solution to this problem which has been proposed in the case of disposable syringes involves the use of a return spring which automatically retracts the needle after a main spring has driven the needle out of the syringe housing. This solution, however, requires a delicate balancing of spring forces so as to ensure that the injection is properly administered. This balancing is not readily achieved at least in a mass-produced, disposable syringe designed for only a single use.

Another proposed solution to this problem involves retraction of the needle, which at least partially resets, or recocks the syringe, but this is not desirable with the disposable unit because it enables the syringe to be reused possibly for illicit purposes.

USA specification no. 3 893 608 discloses a syringe having a housing for containing an ampoule of medication and a hypodermic needle which in an injecting position thereof protrudes from the housing through a needle port. After use of the syringe a hollow piston rod thereof is removed, reversed and engaged over the protruding needle and by axial and rotational movement thereof caused to bend and break the needle and contain the broken-off needle therein.

According to the present invention there is provided a hypodermic syringe comprising:

a housing for containing an ampoule of medication and a hypodermic needle, said needle having an injection position wherein said needle, in use, protrudes from said housing through a needle port; and

deforming and covering means movably mounted on said housing, said deforming and covering means being movable to engage and bend a distal portion of said needle to move said distal portion of said needle from its protruding injecting position to a safe position wherein said distal portion of said needle is covered over by a portion of said deforming and covering means, characterised by said deforming and covering means being disposed on the end of said housing containing said needle port and having a through passage which is coaxial with said needle port when said deforming and covering means is in a first position, the edge of said through passage engaging said protruding needle when said deforming and covering means are moved to a second position wherein the distal portion of said needle is covered by said deforming and covering means.

Thus the needle can be deformed by bending it and covered so that the needle cannot be reused or even separated from the syringe easily. Thus, the needle presents no possible injury problem, nor can it be in any way used for possibly illicit purposes after the syringe has been used for its intended purpose. Moreover, the deforming and covering means can be inexpensively made, and need not appreciably increase the size or weight of the syringe.

The invention will now be described by way of example with reference to the accompanying drawing, in which:

Figure 1 is a side elevational view of an embodiment of disposable hypodermic syringe according to the invention;

Figure 2 is an axial sectional view of the syringe of Figure 1 showing details of needle deforming and covering means thereof, the syringe being shown in cocked, ready-to-use condition;

Figure 3 is an axial sectional view similar to Figure 2, but showing the syringe as it appears after an injection has been administered, and the needle has been driven to a position wherein it protrudes from the syringe housing;

Figure 4 is an axial sectional view similar to Figure 3, but showing the initial phase of actuation of the needle deforming and covering means of the syringe; and

Figure 5 is an axial sectional view similar to Figure 4 showing the final stage of operation of the needle retracting portion of the syringe, wherein the syringe is ready to discard.

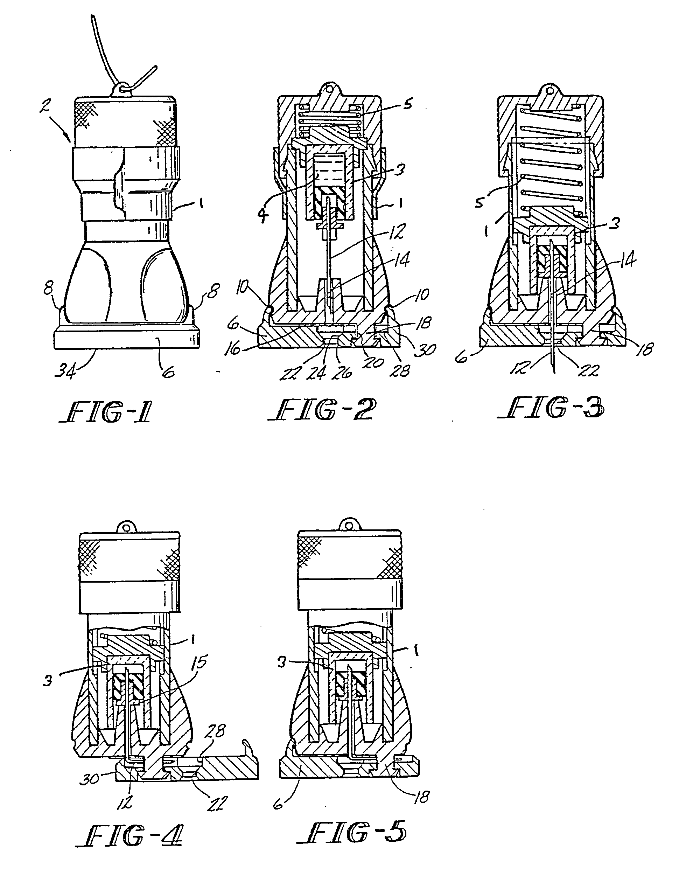

Referring to Figures 1 and 2, there is shown an automatic, disposable hypodermic syringe 2 comprising a housing 1 for containing an ampoule 3 of medicament 4, a hypodermic needle 12, and a spring 5 for driving the needle 12 to an operative position and discharging the medicament 4 therethrough.

The parts of the syringe 2 of particular relevance to the invention are those associated with the needle 12, and these will be described in detail. An end surface 34 of the syringe 2 can be termed the bottom surface, that is, the surface which is pressed against the skin of the user, when administering the injection. The bottom surface 34 is formed on a disc 6 which is movably mounted on the housing 1 of the syringe 2. A pair of tabs 8 engage recesses 10 (Figure 2) in the housing 1 to assist in properly indexing and retaining the disc 6 in the required orientation with respect to the housing 1.

As shown in Figure 2, the syringe 2 has the retracted needle 12 aligned with a bore 14 through which the needle 12 is driven to administer the injection. A strip of tape 16 closes the bore 14 to preserve the sterility of the interior parts of the syringe 2, the tape 16 being pierced when the needle 12 is driven to the protruding position to administer the injection. The lower portion of the housing 1 of the syringe 2 is formed with a projecting post 18 which extends through a hole 20 in the disc 6. Thus the disc 6 is able to pivot about the post 18 with respect to the housing 1; however, the disc 6 is indexed and held in the position shown in Figures 1 and 2 by the tabs 8. The disc 6 also includes a through passage 22 which is coaxial with the needle 12 and the bore 14 when the disc 6 is indexed to the position shown in Figure 2. The passage 22 is provided with chamfered ends 24 and 26 to help prevent snagging of the needle 12 when it is deformed and covered after use. The upper surface of the disc 6 is formed with a closed circular slot 28 which extends radially but eccentrically from the passage 22, stopping short of the side wall 30 of the disc 6. It will be noted that the post 18 extends through the slot 28 which is wider than the post 18 so as not to inhibit rotation of the disc 6 with respect to the housing 1.

It will be appreciated that, with the parts in their respective positions shown in Figure 2, the syringe 2 is in a cocked and ready-to-use condition. On administering an injection, the parts of the syringe 2 move to the respective positions shown in Figure 3, wherein the needle 12 protrudes from the syringe 2 a predetermined distance through the bore 14 and the passage 22. After the injection has been administered, the needle 12 is deformed and covered by turning the disc 6 through a 360° revolution about the post 18, from the position shown in Figure 3 via the position shown in Figure 4 to the position shown in Figure 5. It will, of course, be noted that the axis of rotation, that is the axis of the post 18 and the hole 20, of the disc 6 is radially offset from the axis of the needle 12 and the passage 22. Thus when the disc 6 is rotated about the post 18, the passage 22 is swept along an arcuate path and the wall of the passage 22 bears against the protruding needle 12 and bends it. Since the needle 12 cannot be pulled through the bore 14 due to an enlarged ferrule 15 secured to the needle 12, movement of the passage 22 through this arcuate path will cause the needle 12 to be drawn back through the passage 22 and preferably into the slot 28, as shown in Figure 4. It will be noted that the substantially circular shape of the slot 28 ensures that, during rotation of the disc 6, the outer edge of the slot 28 remain below the bore 14 so that the needle 12 can be smoothly fed into the slot 28 through the passage 22. Engagement of the needle 12 by the passage 22 and the side wall of the slot 28 will bend the needle 12 and render it un-reusable. If the needle 12 is long enough, rotation of the disc 6 back to the position shown in Figure 5, will preferably wrap the needle 12 to some extent around the post 18. It will be noted that only a single tab 8 could be used, or the tabs 8, if two are used, can be break-away tabs so as not to interfere with rotation of the disc 6.

It will be readily appreciated that the bent needle 12 is completely covered, so that it cannot cause harm to anyone. The deforming and covering means formed by the disc 6 is part of the syringe 2, so that it cannot be left behind when the syringe 2 is carried about prior to use. The syringe housing part, which includes the post 18 is preferably made of injection moulded plastics material, as is the disc 6. The disc 6 can simply be press-fitted onto the post 18 and the tabs 8 properly aligned when the syringe 2 is assembled.

Although the embodiment described is a disposable hypodermic syringe, it will be understood that the invention can readily be applied to a non-disposable hypodermic syringe. For example, in such a case the construction may be modified so that the disc 6 can be removed at some convenient time, after use of the syringe 2 and deforming and covering of the needle 12, to permit discarding of the used, bent needle 12 and fitting of a new needle, or preferably a complete new unit comprising the combination of a new ampoule 3 of medication 4 and a new needle 12, for which purpose the housing 1 may be formed of parts which can be disassembled. The disc is then refitted and the syringe is ready for re-use.

## Claims

1. A hypodermic syringe (2) comprising:
a housing (1) for containing an ampoule (3) of medication (4) and a hypodermic needle (12), said needle (12) having an injecting position wherein said needle (12), in use, protrudes from said housing (1) through a needle port (14); and
deforming and covering means (6) movably mounted on said housing (1), said deforming and covering means (6) being movable to engage and bend a distal portion of said needle (12) to move said distal portion of said needle (12) from its protruding injecting position to a safe position wherein said distal portion of said needle (12) is covered over by a portion of said defoming and covering means (6), characterised by said deforming and covering means (6) being disposed on the end of said housing containing said needle port (14) and having a through passage (22) which is coaxial with said needle port (14) when said deforming and covering means (6) is in a first position, the edge of said through passage (22) engaging said protruding needle (12) when said deforming and covering means (6) are moved to a second position so as to move said distal portion of said needle (12) to said safe position wherein the distal portion of said needle is covered by said deforming and covering means (6).

2. A syringe according to claim 1, characterised in that it is of disposable form intended to be used only once, wherein:

said housing (1) contains a said ampoule (3) and a said needle (12), said needle (12) being disposed in a retracted position completely within the confines of said housing (1);

means (5) is provided in said housing (1) for driving said needle (12) through said needle port (14) in said housing (1) to said injecting position; and

movement of said deforming and covering means (6) from said first position to said second position causes it to move laterally across said needle port (14) to engage said needle (12) as it protrudes through said needle port (14) and to bend said needle (12) and pull said needle (12) to said safe position.

3. A syringe according to claim 2, characterised in that said deforming and covering means is a disc (6) mounted adjacent to said one end of said housing (1), said disc (6) being movable across said one end of said housing (1) but being secured to said housing (1) so as not to be readily detachable therefrom, said disc (6) being movable from said first position wherein said needle port (14) is unblocked for passage of said needle (12) therethrough to said second position wherein said disc (6) moves laterally across said needle port (14) to engage the protruding needle (12) and bend the latter thereby retracting said needle (12) out of its protruding position to said safe position wherein said needle (12) is completely covered by a portion of said disc (6).

4. A syringe according to claim 3, characterised in that said disc (6) is mounted on said one end of said housing (1) for rotation about an axis which is radially offset from the axis of said needle port (14).

5. A syringe according to claim 4, characterised in that said disc (6) is provided with an internal slot (28) into which said through passage (22) opens, said slot (28) providing means for containing the bent needle (12).

6. A syringe according to claim 4, characterised by detent means (6, 10) for releasably retaining said disc (6) in said first position.

7. A syringe according to any one of claims 2 to 6, characterised by a tape (16) secured to said one end of said housing (1) overlying said needle port (14) to maintain sterility in the interior of said housing (1) until said tape (16) is pierced by said needle (12) as said needle (12) is driven to said injecting position.

**Patentansprüche**

1. Hypodermatische Spritze (2) mit einem Gehäuse (1) für die Aufnahme einer Ampulle (3) mit Arzneimittel (4) und einer hypodermatischen Nadel (12), wobei diese Nadel (12) eine Injektionsstellung besitzt, in der diese Nadel (12) bei der Verwendung aus dem Gehäuse (1) durch eine Nadelöffnung (14) herausragt, und einer Verformungs- und Abdeckeinrichtung (6), die bewegbar auf dem Gehäuse (1) befestigt ist, wobei die Verformungs- und Abdeckeinrichtung (6) bewegbar ist, so daß sie einen Endabschnitt dieser Nadel (12) ergreift und umbiegt, um diesen Endabschnitt der Nadel (12) aus ihrer hervortretenden Injektionsstellung in eine Sicherheitsstellung zu bewegen, in der dieser Endabschnitt der Nadel (12) von einem Teil der Verformungs- und Abdeckeinrichtung (6) überdeckt ist, dadurch gekennzeichnet, daß die Verformungs- und Abdeckeinrichtung (6) an dem Ende des Gehäuses angeordnet ist, das die Nadelöffnung (14) enthält und einen Durchgang (22) aufweist, welcher koaxial mit der Nadelöffnung (14) ist, wenn die Verformungs- und abdeckeinrichtung (6) sich in einer ersten Stellung befindet, und die Kante dieses Durchgangs (22) die hervortretende Nadel (12) erfaßt, wenn die Verformungs- und Abdeckeinrichtung (6) zu einer zweiten Stellung bewegt wird, um den Endabschnitt der Nadel (12) in die Sicherheitsstellung zu bewegen, in der der Endabschnitt der Nadel von der Verformungs- und Abdeckeinrichtung (6) bedeckt ist.

2. Spritze nach Anspruch 1, dadurch gekenzeichnet, daß sie von wegwerfbarer Form ist, die für einmalige Verwendung bestimmt ist, worin das Gehäuse (1) eine besagte Ampulle (3) und eine besagte Nadel (12) enthält, wobei die Nadel (12) in einer zurückgezogenen Stellung vollständig innerhalb des Gehäuses (1) eingeschlossen ist, eine Einrichtung (5) in dem Gehäuse (1) zum Austreiben der Nadel (12) durch die Nadelöffnung (14) in dem Gehäuse (1) in die Injektionsstellung vorgesehen ist und die Bewegung der Verformungs- und Abdeckeinrichtung (6) aus der ersten Stellung in die zweite Stellung ihre Bewegung seitlich quer zur Nadelöffnung (14) verursacht, um die Nadel (12), wenn sie durch die Nadelöffnung (14) hervorragt, zu ergreifen und die Nadel (12) umzubiegen und die Nadel (12) in ihre Sicherheitsstellung zu ziehen.

3. Spritze nach Anspruch 2, dadurch gekennzeichnet, daß die Verformungs- und Abdeckeinrichtung eine Scheibe (6) ist, die nahe dem einen Ende des Gehäuses (1) befestigt ist, wobei diese Scheibe (6) quer zu dem einen Ende des Gehäuses (1) bewegbar, aber an dem Gehäuse (1) derart befestigt ist, daß sie von ihm nicht leicht entfernbar ist, die Scheibe (6) aus ihrer ersten Stellung, in der die Nadelöffnung (14) für den Durchtritt der Nadel (12) unblokkiert ist, in die zweite Stellung, in der die Scheibe (6) sich seitlich quer zu der Nadelöffnung (14) bewegt, bewegbar ist, um die hervortretende Nadel (12) zu erfassen, und sie umzubiegen und dabei die Nadel (12) aus ihrer hervortretenden Stellung in die Sicherheitsstellung zurückzuziehen, in der die Nadel (12) vollständig von einem Teil der Scheibe (6) bedeckt ist.

4. Spritze nach Anspruch 3, dadurch gekennzeichnet, daß die Scheibe (6) an dem einen Ende des Gehäuses (1) für eine Drehung um eine Achse, die radial von der Achse der Nadelöffnung (14) entfernt ist, befestigt ist.

5. Spritze nach Anspruch 4, dadurch gekennzeichnet, daß die Scheibe (6) mit einem inneren Schlitz (28) versehen ist, in welchen der Durchgang (22) mündet, wobei dieser Schlitz (28) ein Mittel zur Aufname der umgebogenen Nadel (12) darstellt.

6. Spritze nach Anspruch 4, gekennzeichnet durch eine Feststelleinrichtung (6, 10) zur lösbaren Halterung der Scheibe (6) in ihrer ersten Stellung.

7. Spritze nach einem der Ansprüche 2 bis 6, gekennzeichnet durch einen an dem einen Ende des Gehäuses (1) befestigten Streifen (16), der über der Nadelöffnung (14) liegt, um die Sterilität im Inneren des Gehäuses (1) aufrechtzuerhalten, bis der Streifen (16) von der Nadel (12) durchdrungen wird, wenn die Nadel (12) in die Injektionsstellung getrieben wird.

## Revendications

1. Seringue hypodermique (2) comprenant:
une enveloppe (1) pour contenir une ampoule (3) de médication (4) et une aiguille hypodermique (12), ladite aiguille (12) ayant une position d'injection dans laquelle elle fait saillie, en service, de ladite enveloppe (1) à travers un orifice à aiguille (14); et
des moyens de déformation et de recouvrement (6) montés mobiles sur ladite enveloppe (1), lesdits moyens de déformation et de recouvrement (6) étant mobiles pour porter contre un tronçon distal de ladite aiguille (12) et la couder pour amener ledit tronçon distal de ladite aiguille (12) de sa position d'injection saillante dans une position de sécurité dans laquelle ledit tronçon distal de ladite aiguille (12) est recouvert par une partie desdits moyens de déformation et de recouvrement (6), caractérisée en ce que lesdits moyens de déformation et de recouvrement (6) sont disposés sur l'extrémité de ladite enveloppe comportant ledit orifice à aiguille (14) et a un passage traversant (22) qui est coaxial audit orifice à aiguille (14) quand lesdits moyens de déformation et de recouvrement (6) sont dans une première position, le bord dudit passage traversant (22) portant contre ladite aiguille saillante (12) quand lesdits moyens de déformation et de recouvrement (6) sont amenés dans une seconde position afin d'amener ledit tronçon distal de ladite aiguille (12) dans ladite position de sécurité dans laquelle le tronçon distal de ladite aiguille est recouvert par ledit moyen de déformation et de recouvrement (6).

2. Seringue selon la revendication 1, caractérisée en ce qu'elle est de forme à jeter après usage destinée à ne servir qu'une seule fois, et en ce que:

ladite enveloppe (1) contient une susdite ampoule (3) et une susdite aiguille (12), ladite aiguille (12) étant disposée dans une position rétractée entièrement à l'intérieur des confins de ladite enveloppe (1);
un moyen (5) est prévu dans ladite enveloppe (1) pour entraîner ladite aiguille (12) à travers ledit orifice à aiguille (14) de ladite enveloppe (1) jusqu'en ladite position d'injection; et
le déplacement desdits moyens de déformation et de recouvrement (6) de ladite première position jusqu'en ladite seconde position le fait mouvoir latéralement au travers dudit orifice à aiguille (14) pour porter contre ladite aiguille (12) lorsqu'elle fait saillie à travers ledit orifice à aiguille (14) et couder ladite aiguille (12) et attirer ladite aiguille (12) dans ladite position de sécurité.

3. Seringue selon la revendication 2, caractérisée en ce que lesdits moyens de déformation et de recouvrement sont un disque (6) monté près de ladite extrémité de ladite enveloppe (1), ledit disque (6) étant mobile en travers de ladite extrémité de ladite enveloppe (1) mais étant fixé à ladite enveloppe (1) de façon à ne pas pouvoir s'en détacher facilement, ledit disque (6) étant mobile de ladite première position dans laquelle ledit orifice à aiguille (14) est dégagé pour être traversé par ladite aiguille (12) jusqu'en ladite seconde position dans laquelle ledit disque (6) se meut latéralement au travers dudit orifice à aiguille (14) pour porter contre l'aiguille saillante (12) et la couder retirant ainsi ladite aiguille (12) de sa position saillante vers ladite position de sécurité dans laquelle ladite aiguille (12) est entièrement recouverte par une partie dudit disque (6).

4. Seringue selon la revendication 3, caractérisée en ce que ledit disque (6) est monté sur ladite première extrémité de ladite enveloppe (1) de façon à tourner autour d'un axe qui est décalé radialement par rapport à l'axe dudit orifice à aiguille (14).

5. Seringue selon la revendication 4, caractérisée en ce que ledit disque (6) présente une rainure interne (28) dans laquelle débouche ledit passage traversant (22), ladite rainure (28) constituant un moyen propre à contenir l'aiguille coudée (12).

6. Seringue selon la revendication 4, caractérisée par des dispositifs d'arrêt (6, 10) propres à retenir de manière libérable ledit disque (6) dans ladite première position.

7. Seringue selon l'une quelconque des revendications 2 à 6, caractérisée par une bande (16) fixée à ladite extrémité de ladite enveloppe (1) et recouvrant ledit orifice à aiguille (14) pour maintenir stérile l'intérieur de ladite enveloppe (1) jusqu'à être percée par ladite aiguille (12) lorsque ladite aiguille (12) est amenée dans ladite position d'injection.

FIG-1

FIG-2

FIG-3

FIG-4

FIG-5